# FASCICULE DE BREVET EUROPEEN

(11) **EP 3 285 784 B1**
(45) Date de publication et mention de la délivrance du brevet: **08.07.2020**
(21) Numéro de dépôt: 16723433.5
(22) Date de dépôt: 22.04.2016
(51) Int. Cl.: A61K 35/38, A61K 35/741, A61K 35/74, A61K 47/26, A61P 1/00, A61K 9/19, A61K 9/107

(54) **PROCÉDÉ DE PRÉPARATION D'UN ÉCHANTILLON DE MICROBIOTE FÉCAL**
VERFAHREN ZUR HERSTELLUNG EINER FÄKALEN MIKROBIOTAPROBE
METHOD FOR PREPARING A FECAL MICROBIOTA SAMPLE

(30) Priorité: 24.04.2015 FR 1553716
(43) Date de publication de la demande: 28.02.2018
(73) Titulaire: Maat Pharma, 69007 Lyon (FR); Institut national de recherche pour l'agriculture, l'alimentation et l'environnement, 75007 Paris (FR)
(72) Inventeur: AFFAGARD, Hervé, 69007 Lyon (FR); SCHWINTNER, Carole, 69007 Lyon (FR); JUSTE, Catherine, 78610 Le Perray-en-yvelines (FR); DORE, Joël, 94400 Vitry Sur Seine (FR); LEPAGE, Patricia, 75014 Paris (FR); MAILLET, Christel, 91400 Saclay (FR); RABOT, Sylvie, 78720 Senlisse (FR); FONSECA, Fernanda, 78450 Villepreux (FR); BLOTTIERE, Hervé, 44100 Nantes (FR)
(74) Mandataire: Santarelli
(86) Numéro de dépôt international: PCT/FR2016/050958
(87) Numéro de publication internationale: WO 2016/170285

(56) Documents cités:
- WO-A1-2012/122478
- US-A1- 2014 363 398
- US-A1- 2015 037 285
- HAMILTON MATTHEW J ET AL: "Standardized Frozen Preparation for Transplantation of Fecal Microbiota for Recurrent Clostridium difficile Infection", AMERICAN JOURNAL OF GASTROENTEROLOGY, ELSEVIER SCIENCE INC, US, vol. 107, no. 5, 1 mai 2012 (2012-05-01), pages 761-767, XP009161359, ISSN: 0002-9270

## Description

La présente invention se rapporte à un procédé de préparation d'un échantillon de microbiote fécal. L'invention se rapporte également à l'utilisation dudit échantillon dans la transplantation de microbiote fécal, de préférence pour traiter les dysbioses intestinales, notamment les infections à *Clostridium difficile.*

Le microbiote intestinal humain est l'ensemble des micro-organismes (bactéries, levures et champignons) qui se trouvent dans le système gastro-intestinal humain (estomac, intestin et côlon). La diversité microbienne est estimée à l'heure actuelle à environ 10³ espèces bactériennes composant le microbiote intestinal dominant d'un individu adulte, avec une abondance de 10¹⁴ bactéries, représentant un métagénome bactérien de 200 000 à 800 000 gènes chez chaque individu, soit 10 à 50 fois le nombre de gènes du génome humain.

Stérile *in utero,* l'intestin se colonise dès les premiers jours de vie jusqu'à évoluer vers un microbiote individuel unique. Chaque personne possède des bactéries relativement proches en termes d'espèces, mais la composition exacte de son microbiote (espèces, proportions) est pour une large part (plus de ²/₃ des espèces) spécifique de l'hôte.
Ainsi, le microbiote intestinal humain est un écosystème très diversifié, complexe et spécifique de chaque individu.

Il est essentiel pour la santé d'un individu de maintenir un microbiote stable qui est à la fois capable de revenir à son état initial après un changement et résistant à l'invasion. Le maintien d'une grande diversité du microbiote favorise sa stabilité. Cependant, certaines pathologies ou traitements déséquilibrent le microbiote : les antibiotiques par exemple, ainsi que les maladies à composante inflammatoire, telle que les maladies inflammatoires chroniques de l'intestin (MICI), peuvent limiter la diversité du microbiote dans l'intestin. Les traitements antibiotiques (ou antibiothérapie), notamment, résultent en une altération du microbiote, ce qui peut favoriser la prolifération d'organismes pathogènes comme *Clostridium difficile.*

Les infections à *Clostridium difficile* sont responsables de diarrhées nosocomiales ; cette bactérie est résistante à l'antibiothérapie classique (à spectre large, tels que vancomycine ou métronidazole). Afin de rétablir le microbiote intestinal, et lutter contre les infections de type *Clostridium difficile,* et ainsi rétablir l'homéostasie (*i.e.* la symbiose), la transplantation de microbiote fécal est envisagée et testée. Elle consiste en l'introduction des selles d'un sujet donneur sain dans le tube digestif d'un patient receveur, afin de rééquilibrer le microbiote intestinal altérée de l'hôte. Cette transplantation de microbiote fécal peut être allogénique (c'est-à-dire d'un individu donneur sain vers un patient) ou autologue (c'est-à-dire d'un individu vers lui-même). Les résultats obtenus sur les infections de type *Clostridium difficile* sont encourageants, et certains patients ont été traités avec succès (Tauxe et al, Lab Medicine, Winter 2015, volume 46, Number 1).

Cependant, la méthode de transplantation actuelle est empirique et ne prend aucune précaution particulière pour préserver au mieux la viabilité des bactéries anaérobies, composants majoritaires du microbiote intestinal. En outre, l'efficacité de la transplantation de microbiote fécal est variable, et peut nécessiter plus d'une cure. Par ailleurs, la transplantation allogénique nécessite de tester les fèces du donneur pour s'assurer qu'aucun germe pathogène ne sera transplanté au receveur, ou présentera un risque pour le personnel le manipulant pendant l'opération.

Il existe donc un besoin de disposer d'un procédé de transplantation de microbiote fécal qui soit sécurisé, efficace et facile à mettre en œuvre, notamment à l'échelle industrielle. En outre, il existe un besoin pour un procédé de transplantation de microbiote fécal dans lequel la viabilité des bactéries est conservée.

La présente invention permet de répondre à ces besoins.

La présente invention se rapporte donc à un procédé de préparation d'un échantillon de microbiote fécal d'un sujet donneur, comprenant les étapes suivantes:
a) le prélèvement d'au moins un échantillon de microbiote fécal du sujet donneur,
b) dans un délai inférieur à 5 minutes suivant le prélèvement, le placement dudit échantillon obtenu en a) dans un dispositif de collecte étanche à l'oxygène,
c) le mélange de l'échantillon obtenu en b) avec au moins une solution aqueuse saline comprenant au moins un cryoprotectant et un agent de charge,
d) optionnellement, la filtration du mélange obtenu en c), notamment par un filtre comprenant des pores de diamètre inférieur ou égal à 0.7 mm, de préférence inférieur ou égal à 0.5 mm et
e) le stockage du mélange obtenu en c) ou d) par congélation à une température comprise entre -15°C et -100°C, de préférence entre -60°C et -90°C,
les étapes b) à e) étant réalisées en anaérobiose.

Un tel procédé de transplantation de microbiote fécal est en effet facile à mettre en œuvre, et son efficacité peut être estimée en comparant la population microbienne obtenue après le procédé, comparée au prélèvement initial. Différents indices peuvent être utilisés pour évaluer cette efficacité, et les résultats ci-dessous ont pu être obtenus :

| **Indices** | **Dissimilarité de Bray-Curtis** | **Distance de Canberra** | **Indice de Jaccard** | **Divergence de Jensen-Shannon** | **Indice de Morisita** | **Coefficient de corrélation de Pearson** |
|---|---|---|---|---|---|---|
| Famille | <0,4 | <0,7 | <0,6 | <0,4 | <0,5 | >0,7 |
| Genre | <0,5 | <0,7 | <0,7 | <0,4 | <0,6 | >0,5 |
| OTU | <0,7 | <0,9 | <0,8 | <0,4 | <0,7 | >0,3 |

La présente invention se rapporte également à l'utilisation d'un échantillon de microbiote fécal d'un sujet donneur susceptible d'être obtenu par le procédé selon l'invention, à l'état décongelé, dans la transplantation de microbiote fécal autologue ou allogénique.

La présente invention se rapporte également à l'utilisation d'un échantillon de microbiote fécal d'un sujet donneur susceptible d'être obtenu par le procédé selon l'invention, à l'état décongelé, pour traiter les dysbioses intestinales, et notamment les infections à *Clostridium difficile,* les dysbioses induites par des traitements médicamenteux, par des traitements physiques (radiations notamment), par des interventions chirurgicales (intestinales notamment), ou par des apports nutritionnels. La présente invention se rapporte également à l'utilisation d'un échantillon de microbiote fécal d'un sujet donneur susceptible d'être obtenu par le procédé selon l'invention, à l'état décongelé, pour traiter une pathologie choisie parmi les maladies inflammatoires chroniques de l'intestin (MICI), les troubles fonctionnels intestinaux, l'obésité, les maladies métaboliques (diabète de type 2, syndrome métabolique notamment) et les maladies auto-immunes (diabète de type 1, notamment), les allergies, les maladies hépatiques (stéatose, cirrhose notamment), certaines maladies neurologiques (autisme notamment) et certains cancers (cancer colorectal notamment).
Par dysbiose intestinale, on entend tout déséquilibre soutenu du microbiote intestinal. Par déséquilibre soutenu du microbiote intestinal, on entend toute perte de micro-organismes bénéfiques, et/ou toute perte de diversité de micro-organismes, et/ou toute expansion ou développement de micro-organismes agressifs parmi les commensaux (pathobiontes), et/ou toute prolifération de micro-organismes pathogènes (*C. difficile* notamment). Toute altération soutenue du microbiote intestinal humain peut en effet engendrer un état pathologique. Notamment, la réduction de la diversité au sein du microbiote est caractéristique des maladies associées à une dysbiose (obésité, maladie de Crohn, diabète ou allergie notamment) (Sansonetti, Collège de France, 22 janvier 2014).
De préférence, la pathologie à traiter est une dysbiose intestinale.
Par maladies inflammatoires chroniques de l'intestin (MICI), on entend notamment la maladie de Crohn, la rectocolite hémorragique.
Par troubles fonctionnels intestinaux, on entend notamment le syndrome du côlon irritable, la colite spasmodique.

Le procédé de préparation d'un échantillon de microbiote fécal d'un sujet donneur selon l'invention comprend ainsi une étape a) de prélèvement d'au moins un échantillon de microbiote fécal du sujet donneur.
Cette étape se fait de préférence par prélèvement d'un échantillon de selles du sujet donneur. En effet, l'échantillon de selles contient du microbiote fécal du sujet donneur. Ainsi, le procédé selon l'invention comprend une étape a) de prélèvement d'au moins un échantillon de selles, comprenant le microbiote fécal, du sujet donneur.
De préférence, selon l'invention le sujet donneur est un sujet humain sain. Par sujet « sain », on entend un sujet non atteint d'un déséquilibre du microbiote intestinal ou d'une pathologie diagnostiquée/reconnue par le corps médical.
De préférence, l'échantillon de selles présente une masse d'au moins 20g.

Suite à cette étape de prélèvement, et dans un délai très rapide, *i.e.* inférieur à 5 minutes suivant le prélèvement, de préférence inférieur à 3 minutes, plus préférentiellement inférieur à 1 minute, le prélèvement obtenu en a) est placé dans un dispositif de collecte étanche à l'oxygène : c'est l'étape b).
Toute la suite du procédé s'effectue désormais en anaérobiose (*i.e.* en atmosphère anaérobie).

De préférence, le dispositif de collecte étanche à l'air se présente sous une forme du type comprenant :
- un conteneur comprenant un corps qui comporte un espace intérieur adapté pour recevoir l'échantillon de microbiote fécal du sujet donneur, et un col qui délimite une ouverture d'accès à l'espace intérieur du corps, et
- un couvercle adapté pour être monté de manière amovible et étanche sur le col du conteneur de manière à obturer l'ouverture d'accès du col et à fermer l'espace intérieur du corps,
dans lequel le corps du conteneur est constitué d'une poche souple, et dans lequel au moins l'un parmi le conteneur et le couvercle est pourvu d'un organe d'évacuation adapté pour évacuer au moins une partie des gaz contenus dans l'espace intérieur du corps du conteneur.
De préférence, l'organe d'évacuation du dispositif comprend un passage ménagé au travers de l'un parmi le conteneur et le couvercle, et un élément d'obturation du passage pour empêcher des fluides extérieurs de pénétrer dans l'espace intérieur du corps du conteneur. De préférence, l'organe d'évacuation du dispositif comprend en outre une membrane de filtration microporeuse disposée dans le passage.
Alternativement, le dispositif de collecte étanche à l'air se présente sous une forme du type comprenant :
- un conteneur comprenant un corps qui comporte un espace intérieur adapté pour recevoir l'échantillon de microbiote fécal du sujet donneur, et un col qui délimite une ouverture d'accès à l'espace intérieur du corps, et
- un couvercle adapté pour être monté de manière amovible et étanche sur le col du conteneur de manière à obturer l'ouverture d'accès du col et à fermer l'espace intérieur du corps,
dans lequel l'espace intérieur du corps du conteneur comprend éventuellement un dispositif chimique neutralisant l'oxygène.
De préférence, le dispositif de collecte étanche à l'air est utilisé pour les étapes a) et b) : le prélèvement de l'échantillon de l'étape a) s'effectue directement dans ledit dispositif, notamment dans le conteneur, et la fermeture du dispositif, notamment grâce au couvercle, place l'échantillon en atmosphère sans oxygène (étape b).

Selon le procédé de l'invention, les étapes b) à e) sont réalisées sous une atmosphère dépourvue d'oxygène. En anaérobiose, la viabilité des bactéries constitutives du microbiote fécal et présentes dans l'échantillon est ainsi préservée.
En particulier, le dispositif utilisé à l'étape b), mentionné ci-dessus, permet de réaliser toutes les étapes b) à e) en anaérobiose.

Une fois l'échantillon (obtenu en a) placé dans un dispositif de collecte étanche à l'oxygène, il peut, de façon optionnelle, être incubé à une température comprise entre 33°C et 40°C pendant une durée maximale de 75h. De préférence, cette étape d'incubation se fait à une température comprise entre 35°C et 38°C, pendant une durée comprise entre 24h et 73h. Idéalement, cette étape se fait à une température d'environ 37°C pendant 72h. Alternativement, l'échantillon peut, de façon optionnelle, être incubé à une température comprise entre 2°C et 10°C pendant une durée maximale de 75h. De préférence, cette étape d'incubation se fait à une température comprise entre 4°C et 8°C, pendant une durée comprise entre 24h et 72h.
A l'issue de cette étape, un contrôle visuel peut être effectué, afin d'évaluer la qualité de l'échantillon obtenu à ce stade du procédé. Si ce contrôle est conforme, de façon optionnelle, une étape de transport peut ainsi avoir lieu. Cette étape de transport permet de rapatrier l'échantillon du lieu de prélèvement au laboratoire, pour traitement ultérieur et analyse. Le contrôle visuel mentionné précédemment peut également être réalisé après le transport.

Ainsi, de préférence, l'échantillon placé dans le dispositif de collecte de l'étape b) subit une étape de transport avant l'étape c).

De préférence également, l'échantillon placé dans le dispositif de collecte de l'étape b) est incubé à une température comprise entre 33°C et 40°C, de préférence entre 35°C et 38°C, pendant une durée maximale de 75h, de préférence comprise entre 24h et 73h, entre les étapes b) et c). De préférence, l'incubation a lieu avant et pendant l'étape de transport.

Puis intervient l'étape c) : cette étape comprend le mélange de l'échantillon obtenu en b) avec au moins une solution aqueuse saline comprenant au moins un cryoprotectant et un agent de charge.
Si une étape de transport, et éventuellement une étape d'incubation, ont lieu, alors l'étape c) comprend bien évidemment le mélange de l'échantillon obtenu en b), après transport et/ou incubation, avec au moins une solution aqueuse saline comprenant au moins un cryoprotectant et un agent de charge.

Typiquement, la solution aqueuse saline selon l'invention comprend de l'eau et des sels physiologiquement acceptables. Typiquement, les sels sont des sels de calcium, sodium, potassium ou magnésium, avec des ions chlorure, gluconate, acétate ou hydrogénocarbonate.
La solution aqueuse saline selon l'invention peut également optionnellement comprendre au moins un antioxydant. L'antioxydant est notamment choisi parmi l'acide ascorbique et ses sels (ascorbate), les tocophérols (notamment l'a-tocophérol), la cystéine et ses formes salifiées (chlorhydrate notamment) et leurs mélanges.

De préférence, la solution aqueuse saline selon l'invention comprend :
- au moins un sel choisi parmi le chlorure de sodium, le chlorure de calcium, le chlorure de magnésium, le chlorure de potassium, le sodium gluconate et le sodium acétate, et
- optionnellement au moins un antioxydant, de préférence choisi parmi le L-ascorbate de sodium, les tocophérols, le chlorhydrate de L-cystéine monohydraté et leurs mélanges. Typiquement, le sel est présent dans la solution aqueuse saline en concentration comprise entre 5 et 20g/L, de préférence entre 7 et 10 g/L.
Typiquement, l'antioxydant est présent dans la solution aqueuse saline en quantité comprise entre 0.3 et 1% en poids par rapport au volume total de solution, de préférence en quantité comprise entre 0.4 et 0.6% en poids par rapport au volume total de solution. De préférence, lorsque l'antioxydant est un mélange de L-ascorbate de sodium et de chlorhydrate de L-cystéine monohydraté, le L-ascorbate de sodium est présent en quantité comprise entre 0.4 et 0.6% en poids par rapport au volume total de solution, et le chlorhydrate de L-cystéine monohydraté est présent en quantité comprise entre 0.01 et 0.1% en poids par rapport au volume total de solution.

La solution aqueuse saline selon l'invention comprend également au moins un cryoprotectant. Un cryoprotectant est une substance utilisée pour protéger l'échantillon des dommages causés par la congélation, notamment dus à la formation de cristaux de glace.
Le cryoprotectant est choisi parmi le tréhalose et le galactose-lactose et leurs mélanges. Plus préférentiellement, le cryoprotectant est le galactose-lactose ou le tréhalose.

La solution aqueuse saline selon l'invention comprend au moins un agent de charge.
L'agent de charge est de préférence choisi parmi les hydrolysats partiels d'amidon ou de fécule. Les hydrolysats partiels d'amidon, notamment de blé ou de maïs, ainsi que les hydrolysats partiels de fécule, par exemple de pomme de terre, comprennent une forte quantité de maltodextrines. Les maltodextrines sont le résultat de l'hydrolyse partielle d'amidon ou de fécule, et sont constituées de différents sucres (glucose, maltose, maltotriose, oligo- et polysaccharides), dont les proportions varient en fonction du degré d'hydrolyse.
De préférence, l'agent de charge présent dans la solution aqueuse saline est un mélange de maltodextrines, dans lequel la quantité de maltodextrines est comprise entre 4 et 20% en poids par rapport au volume total de solution.

La solution aqueuse saline selon l'invention comprend à la fois :
- au moins un cryoprotectant tel que décrit ci-dessus, i.e. choisi parmi le tréhalose, le galactose-lactose et leurs mélanges, et
- au moins un agent de charge tel que décrit ci-dessus, i.e. choisi parmi les hydrolysats partiels d'amidon ou de fécule, de préférence l'agent de charge est constitué de maltodextrines.
De préférence, dans ce cas, la quantité de cryoprotectant est comprise entre 3 et 30% en poids par rapport au volume total de solution, de préférence entre 4% et 20% en poids par rapport au volume total de solution ; et la quantité d'agent de charge, de préférence de maltodextrines, est comprise entre 4 et 20% en poids par rapport au volume total de solution.
L'étape c) de mélange de l'échantillon obtenu en b) avec au moins une solution aqueuse saline comprenant au moins un cryoprotectant peut notamment être réalisée par malaxage, afin d'obtenir un mélange homogène.

De préférence, l'échantillon obtenu en b) est mélangé avec ladite solution aqueuse saline dans un rapport poids/volume respectif compris entre 0.5 poids : 10 volumes et 2 poids : 2 volumes. Un rapport poids/volume échantillon : solution égal à 0.5 poids : 10 volumes signifie que l'échantillon est mélangé à 0.5 poids (par exemple 0.5g) pour 10 volumes de solution (par exemple 10ml). De préférence, le rapport poids/volume échantillon : solution est égal à 1 poids d'échantillon pour 4 volumes de solution (1 poids : 4 volumes).

Une fois cette étape réalisée, une étape d) optionnelle peut être effectuée. L'étape d) comprend la filtration du mélange obtenu en c), notamment par un filtre comprenant des pores de diamètre inférieur ou égal à 0.7 mm, de préférence inférieur ou égal à 0.5 mm. Une telle filtration permet la rétention des particules grossières, et la récupération des bactéries d'intérêt (constitutives du microbiote fécal) dans le filtrat.

Puis, suite à l'étape c) ou d), lorsque cette dernière a lieu, le mélange obtenu est stocké par congélation à une température comprise entre -15°C et -100°C : c'est l'étape e). De préférence, la température de congélation (et donc de stockage) est comprise entre -60°C et -90°C ; plus préférentiellement elle est d'environ -80°C ou d'environ -65°C.

Afin d'être congelé, suite à l'étape c) ou d), et avant l'étape e), le mélange peut être préalablement aliquoté, pour assurer des spécimens de volume constant. Par exemple, l'aliquotage est effectué pour obtenir des spécimens de volume égal à 50 ml, 100 ml, 150 ml, ou 200 ml. De préférence, l'aliquotage est effectué pour obtenir des spécimens de volume égal à 100 ml.
Cette étape de congélation et stockage permet de conserver les échantillons traités pendant une durée d'au moins 2 mois. Les échantillons ainsi stockés présentent en outre une bonne qualité, même après décongélation.

De préférence, le procédé selon l'invention comprend une étape f) de décongélation de l'échantillon congelé obtenu en e), en anaérobiose, jusqu'à température ambiante. Cette étape f) de décongélation peut s'effectuer en mettant l'échantillon congelé au bain-marie à une température comprise entre 35°C et 40°C, par exemple 37°C, pendant une durée de quelques minutes (typiquement de 2 à 10 minutes). L'étape f) de décongélation peut également s'effectuer en mettant l'échantillon congelé à une température comprise entre 2°C et 10°C, par exemple entre 4°C et 8°C, pendant une durée de 10 à 20 heures.

L'échantillon ainsi décongelé, à température ambiante, peut ensuite être administré au patient receveur.
Le patient receveur peut être différent du sujet donneur, et la transplantation est alors allogénique.
Le patient receveur peut aussi être identique au sujet donneur, et la transplantation est alors autologue ; ce type de transplantation peut avoir lieu lorsque le sujet, alors sain, donne un échantillon avant l'altération de son microbiote. L'échantillon est alors congelé selon les étapes décrites dans la présente demande, puis transplanté à ce même sujet (patient receveur) si celui-ci présente notamment une infection à *Clostridium difficile.* La transplantation de microbiote fécal autologue présente l'avantage d'éviter la transmission d'agent pathogène provenant d'un autre donneur.

La présente invention se rapporte également à un échantillon de microbiote fécal d'un sujet donneur susceptible d'être obtenu par le procédé selon l'invention, pour son utilisation dans la transplantation de microbiote fécal autologue ou allogénique.

La présente invention se rapporte également à un échantillon de microbiote fécal d'un sujet donneur susceptible d'être obtenu par le procédé selon l'invention, pour son utilisation pour traiter les infections à *Clostridium difficile.* La présente invention se rapporte également à un échantillon de microbiote fécal d'un sujet donneur susceptible d'être obtenu par le procédé selon l'invention, pour son utilisation pour traiter une pathologie choisie parmi les maladies inflammatoires chroniques de l'intestin (MICI), les troubles fonctionnels intestinaux, l'obésité, les maladies métaboliques et les maladies auto-immunes, les allergies, les maladies neurologiques et les cancers.

L'invention va maintenant être exemplifiée à l'aide des exemples qui suivent, qui ne sont pas limitatifs.

Les légendes des figures sont les suivantes :

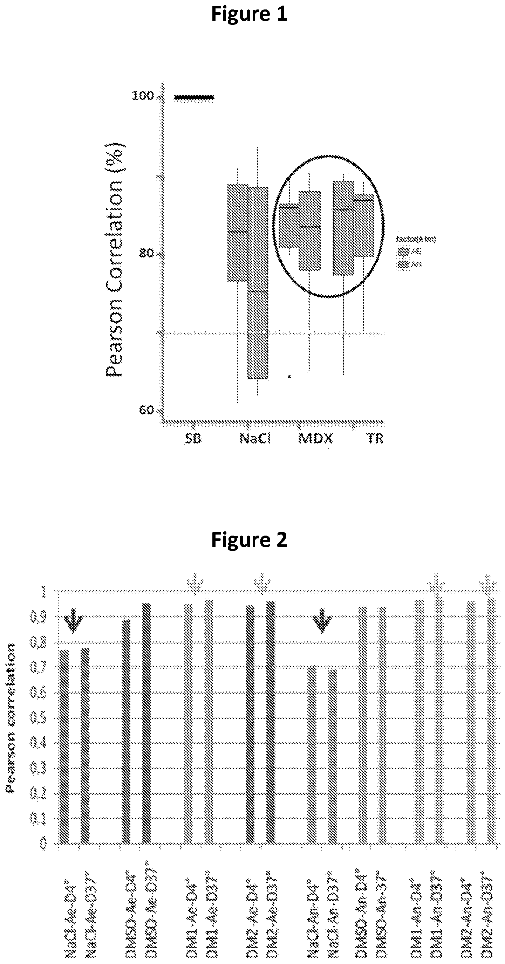

**Figure 1** **: corrélation de Pearson pour les profils de phylo-transcriptomique**
   - SB. Contrôle échantillon fécal brut (non conditionné)
   - NaCl. Solution saline
   - MDX. Saline avec maltodextrines 15% (p/v) + tréhalose 5% (p/v)
   - TR. Saline avec tréhalose 15% (p/v) + maltodextrines 5% (p/v)
   - AE (rouge). Aérobie (exposition à l'air)
   - AN (bleu). Anaérobie (non exposé à l'air + antioxydants)
**Figure 2** **: résultats des analyses métabolomiques**
   DM1 = MDX15
   DM2 = TR15
**Figure 3** **: corrélations de Spearman au niveau des genres bactériens pour les différents diluants testés après une semaine de conservation, comparées au témoin "selles fraîches"**
   DM1 = MDX15
   DM2 = TR15
**Figure 4****: cinétique de colonisation des populations de *Bacteroides* et *Faecalibacterium* dans les fèces de souris**

### Exemple 1: Préparation d'un échantillon de microbiote fécal d'un sujet donneur selon l'invention

### Matériels & Méthodes :

### Prélèvement d'échantillons

Pendant trois jours consécutifs, 3 participants (un par jour) sont invités à fournir un échantillon de selles fraîches, recueilli le matin et mis en anérobiose à l'aide de l'ajout d'un catalyseur type Anaerocult® IS (ref 116819 chez Merck-Millipore) (étape a) du procédé). Une évaluation visuelle et la qualification des selles sont faites selon le tableau de Bristol.
Dans les 2h après la collecte, les selles sont homogénéisées pendant 5 minutes en atmosphère anaérobie, par malaxage manuel dans la poche de collecte (étape b) du procédé). De petits aliquots (150-200mg) de matière fécale brute (SB, pour Selle Brute) sont conservés pour le profilage de l'ADNr 16S et de l'ARNr 16S des matières fécales brutes. Un aliquot (1 g) est dilué dans du bouillon cerveau-cœur enrichi et froid, centrifugé à 220 000xg, à 4°C pendant 1h, et le surnageant est fractionné en aliquots de 1ml pour le profilage métabolomique d'eau fécale brute. Les aliquots pour l'ADN, l'ARN et le profilage métabolomique sont stockés à -80°C. Un troisième aliquot (0,4 g) est dilué dans 1,6ml du bouillon de culture et utilisé pour inoculer 3 tubes de culture Kimax (0,5 ml d'inoculum pour 9,5 ml de bouillon, extemporanément enrichi en L-ascorbate de sodium et en chlorhydrate de L-cystéine monohydrate à une concentration finale de 0.5% [p/v] et 0,05% [p/v], respectivement) pour le test d'activité de référence.
Huit fractions de selles sont ensuite transférées dans des sacs filtrants Stomacher : 4 sacs pour le conditionnement dans les quatre diluants dans des conditions anaérobies, et quatre sacs pour le conditionnement dans les mêmes quatre diluants dans des conditions aérobies.

### Conditionnement

Deux équipes réalisent les étapes de conditionnement en parallèle pour assurer que tous les échantillons sont traités de manière identique.
Les 4 fractions sous chaque condition d'atmosphère sont remises en suspension dans 4 volumes des solutions aqueuses suivantes (étape c) du procédé):
- Solution saline 9g/L (identifiée « NaCl »),
- DMSO 6,25% (v/v) dans une solution saline 9 g/L (identifiée « DMSO »),
- MDX (maltodextrines) 15% (p/v) + TR (tréhalose) 5% (p/v) dans une solution saline 9g/L (identifiée « MDX15 »), et
- MDX 5% + TR 15% dans une solution saline 9g/L (identifiée « TR15 »).
Les préparations MDX15 et TR15 réalisées sous atmosphère anaérobie sont en outre complétées par les deux agents réducteurs, L-ascorbate de sodium et chlorhydrate de L-cystéine monohydraté, à une concentration finale de 0.5% (p/v) et 0,1% (p/v), respectivement.
La remise en suspension est assurée par un mélange manuel pendant 5 minutes à travers le sac. Ce mélange assure la filtration en même temps, à travers une gaze (trous de 0,5 mm) présente dans le sac (étape d) du procédé). 20 ml de chaque filtrat sont ensuite transférés avec une pipette dans deux sacs de congélation CryoMACS® Freezing Bags 50 (Ref Miltenyi Biotec SAS 200-074-400), donnant un total de 16 CryoMACS® pour chaque donneur (2 x NaCl-An, 2 × DMSO-An, 2 × MDX15-An, 2 × TR15-An, 2 x NaCl-Ae, 2 × DMSO-Ae, 2 × MDX15-Ae, 2 × TR15-Ae), qui sont stockés à -80°C (étape e) du procédé).

Des aliquots de chaque filtrat sont également conservés pour l'ADNr 16S, l'ARNr 16S, le profilage métabolomique, et les cultures bactériennes. Pour l'ADNr 16S, 16S ARNr et la métabolomique, 6 aliquots de 1ml de chaque suspension sont centrifugés à 5000 x g, 4°C pendant 30 minutes; les surnageants regroupés sont en outre ultra-centrifugés (220 000 x g, 4°C, 1 h) pour la métabolomique, tandis que le culot humide de × 5000 g est conservé pour le profilage de l'ADNr 16S et de l'ARNr 16S. Trois aliquots de 0,5 ml de chaque filtrat sont utilisés pour inoculer 3 tubes de culture Kimax de bouillon de culture, comme décrit précédemment pour les matières fécales brutes. L'ensemble du processus est répété 3 fois sur 3 jours consécutifs pour gérer 3 selles différentes de trois donneurs différents.

### Reconditionnement

Pour assurer le même temps de stockage pour les échantillons des 3 donneurs, les sacs CryoMACS sont décongelés sur 3 jours consécutifs à raison d'un sac par personne et par jour. La décongélation est effectuée selon deux protocoles différents (étape f) du procédé):
- pendant une nuit à 4°C ;
- pendant 5 minutes à 37°C dans un bain-marie.
Après décongélation, les échantillons sont mis en culture dans un bouillon cerveau-cœur enrichi, et l'activité métabolique est évaluée. Les aliquots de filtrats décongelés avant culture (filtrats décongelés non cultivés) sont également conservés pour l'ADNr 16S, l'ARNr 16S et le profilage métabolomique.

### Les cultures microbiennes

A chaque étape critique du procédé, un échantillon est recueilli et utilisé pour ensemencer des tubes de culture en triplicate contenant chacun 9,5mL de bouillon cerveau-cœur enrichi. Les tubes de culture avaient déjà été réduits dans la chambre anaérobie pour éliminer tout dioxygène dissous et permettre la croissance des bactéries anaérobies strictes.

Après incubation pendant 48 heures à 37°C dans des conditions anaérobies strictes, les cultures en triplicate sont récoltées, regroupées dans des tubes Falcon50, et centrifugées pendant 30 min à 5000 x g, 4°C. Le surnageant est en outre ultra-centrifugé pendant 1 heure à 220 000 xg, 4°C pour le profilage métabolomique (1 ml de surnageant de fraction conservée à -80°C), tandis que le culot humide de 5000 × g est divisé en trois fractions égales dans des tubes Sarstedt pour le 16S ADNr et 16S ARNr, tous les aliquots étant conservés à -80°C jusqu'aux analyses.

### Analyses métabolomiques

Les analyses métabolomiques sont ensuite effectuées, pour obtenir les profils LC-MS (Q-Exactive Thermofisher Scientific) dans les modes d'ionisation positifs et négatifs.

### Profil phylogénétique

L'ADN total est extrait. Il est ensuite contrôlé et séquencé par pyroséquençage.

### Profils transcriptomiques

L'ARN est extrait en utilisant la méthode suivante : brièvement, les bactéries sont lysées par traitement chimique et mécanique ; puis les lysats sont précipités et centrifugés ; enfin l'ARN est isolé et purifié sur minicolonnes en utilisant le kit High Pure Isolation Kit (Roche). Leur intégrité est évaluée et ils sont ensuite soumis à une RT-PCR.
Les ADNc sont séquencés par pyroséquençage, puis soumis à la même analyse que pour l'ADN.

### Résultats :

### Profil phylogénétique

Le facteur discriminant majeur est le sujet. Ceci était attendu, étant donné que la spécificité d'hôte de la flore microbienne est bien établie. Cela signifie que quel que soit l'effet de conditionnement des selles, il respectera la spécificité d'hôte. Les comparaisons effectuées seront donc fiables et les comportements conservés pour différents individus seront plus significatifs.
Les corrélations de Pearson et Spearman ont ensuite été utilisées pour évaluer le diluant de conditionnement le plus efficace. Des observations similaires ont été faites lorsque l'on compare les profils phylogénétiques basés sur l'ADN total et les profils de phylo-transcriptomique basés sur l'ARN. L'illustration ci-dessous (Figure 1) met en évidence les principaux résultats obtenus.

### Impact de la procédure de reconditionnement sur l'intégrité de l'échantillon

Les conditions comparées pour le re-conditionnement des préparations congelées qui doivent être utilisées pour la ré-administration sont :
- pendant une nuit à 4°C ; ou
- pendant 5 minutes à 37°C.
Quel que soit le diluant (MDX15 ou TR15), la décongélation rapide à 37°C apparaît la plus favorable pour la reconstruction d'un microbiote proche des selles initiales.

### Profils transcriptomiques

Les corrélations de Pearson entre les profils de phylo-transcriptomique sont discriminantes et informatives, alors qu'entre les profils de phylo-génomique elles ne le sont pas.

La figure 1 présente les corrélations de Pearson entre les profils de phylo-transcriptomique obtenus à partir de la distribution au niveau des familles bactériennes dans l'ARN total des différentes fractions préparées. La référence est le profil obtenu à partir de matières fécales brutes (SB pour 'Selles Brutes').

La figure indique que des diluants contenant les mélanges de maltodextrine (MDX) et le tréhalose (TR) permettent une meilleure préservation de l'intégrité du microbiote dominant par rapport à la solution saline (NaCl). Depuis, des observations similaires ont été faites pour l'ADN, ce qui signifie que le procédé permet de préserver les populations et leur intégrité fonctionnelle.

### Analyses métabolomiques

Les résultats des analyses métabolomiques sont présentés en Figure 2.

Les profils métaboliques des cultures de suspensions fécales décongelées dans MDX15 ou TR15 sont les plus similaires aux cultures des suspensions fraîches correspondantes (flèches vertes), que ce soit en condition aérobie (Ae) ou anaérobie (An). Des corrélations plus faibles ont été obtenues entre les cultures de suspensions congelées et fraîches en NaCl (flèches rouges). Le reconditionnement à 4°C pendant la nuit ou à 37°C pendant 5 minutes fait peu de différence sur le profil métabolomique. Dans l'ensemble, sur la base de la conservation des profils métaboliques entre les cultures de matières fécales fraîches, des suspensions de matières fécales fraîches et les mêmes suspensions congelées-décongelées, **les maltodextrines ou le tréhalose sont des cryoprotectants et agents de charge très efficaces pour les transplantations fécales.**

### Exemple 2: Reconstruction des microbiotes dans des souris axéniques

La procédure vise à analyser l'impact du processus de conditionnement de selles humaines sur l'écologie intestinale reconstruite après inoculation en souris axéniques de lignée C3H/HeN. L'approche standardisée comporte un groupe témoin et plusieurs groupes tests. Par comparaison au groupe témoin recevant une suspension des selles fraîchement collectées, les groupes tests reçoivent des préparations réalisées à partir des mêmes selles :
- congelées en NaCl,
- congelées avec maltodextrines 15% et tréhalose 5% (« MDX15 » décrits à l'exemple 1) ; ou
- congelées avec tréhalose 15% et maltodextrines 5% (« TR15 » décrits à l'exemple 1). Ces diverses conditions visent à optimiser la préservation du microbiote. Les fractions congelées sont administrées *in vivo* après conditionnement et stockage pendant 1 et 7 semaines. Un témoin d'essai a été réalisé avec la selle brute formulée en NaCl, implantée immédiatement après conditionnement. Cette procédure fait appel à l'utilisation de souris axéniques recevant les différentes préparations d'inoculum *per os,* à l'aide d'une sonde oro-gastrique (0,2 mL/souris). L'évaluation de la réussite de la procédure est basée sur la caractérisation du microbiote en termes de composition et d'activité, la similarité maximale (%) avec le contrôle étant initialement recherchée. Cette procédure est mise en œuvre sur 4 animaux par condition pour tester un ensemble de conditions préalablement validées par des analyses comparées *in vitro.*

***Prélèvements et analyses*:** après 2 jours, 4 jours et 15 jours, 2 à 3 pellets fécaux fraîchement et spontanément émis sont collectés par animal le matin pour analyse microbiologique. Par ailleurs un échantillon de contenu caecal est collecté au sacrifice à 15 jours pour analyse phylogénomique et/ou transcriptomique et métabolomique.

### Résultats :

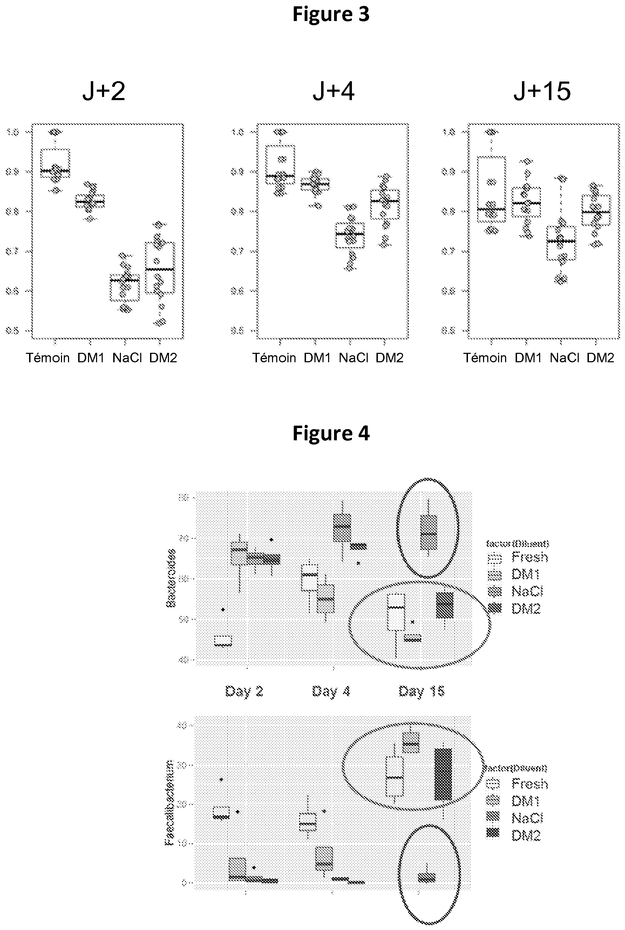

Ces analyses ont été menées sur la première série de cages (1 semaine de conservation vs témoin). Les ADN ont été extraits puis séquencés en rDNA 16S. Les OTU (Operational Taxonomic Unit) ont été identifiées et quantifiées. Une corrélation statistique de Spearman permet ensuite de comparer les contenus taxonomiques de chaque échantillon testé. Les résultats sont présentés en Figure 3.

Les corrélations de Spearman calculées entre les différentes conditions montrent que la colonisation se fait de manière efficace avec un avantage aux formulations DM1 et DM2 comparées avec le NaCl 0,9% seul. A J+2, la formulation DM1 donne des similarités très proches des témoins, alors que la formulation DM2 arrive à un niveau de performance équivalent à J+4. A J+4, le microbiote se stabilise et il y a peu de différence observée avec le point J+15.

Une analyse approfondie au niveau des genres bactériens les plus affectés dans ces essais, c'est-à-dire les genres *Bacteroides* et *Faecalibacterium,* est présentée dans la Figure 4.

L'évolution de D2 à D15 montre bien que le diluant DM1 présente rapidement un profil plus proche de la selle fraîche que le diluant DM2. A 15 jours cependant tous deux ont des profils similaires. *A contrario,* le NaCl favorise une colonisation forte des *Bacteroides*, bactéries pro-inflammatoires, au détriment en particulier des *Faecalibacterium,* qui ne parviennent pratiquement pas à s'implanter. Or le genre *Faecalibacterium* a été beaucoup étudié car présentant des propriétés anti-inflammatoires et concourant à l'efficacité de la barrière intestinale (Everard, 2013; Sokol, 2008). Sa présence en faible quantité dans le microbiote humain est également corrélée avec différentes pathologies (maladie de Crohn, obésité, maladies inflammatoires de l'intestin...).
L'utilisation du NaCl ne permet donc pas de rétablir une flore de même qualité que la selle fraîche initiale, alors que les diluants DM1 et DM2 y parviennent, avec un effet légèrement plus rapide avec DM1, ainsi qu'une variabilité inter-individus plus faible, représentée par la hauteur de la box.

L'évolution de la répartition des familles bactériennes suit une même tendance entre le groupe témoin, le groupe ayant reçu l'échantillon avec MDX15 (DM1) et le groupe ayant reçu l'échantillon avec TR15 (DM2).

La même tendance est observée sur les prélèvements et analyses effectués après 7 semaines de stockage (résultats non montrés).

**Il ressort ainsi que les maltodextrines ou le tréhalose permettent une recolonisation efficace de l'intestin sans altération du microbiote initial.**

Everard, A., 2013. Cross-talk between Akkermansia muciniphila and intestinal epithelium controls diet-induced obesity. Proc Natl Acad Sci U S A, pp. 110(22): 9066-71.

Sokol, H., 2008. Faecalibacterium prausnitzii is an anti-inflammatory commensal bacterium identified by gut microbiota analysis of Crohn disease patients. Proc Natl Acad Sci U S A, pp. 105(43):16731-6.

## Revendications

1. Procédé de préparation d'un échantillon de microbiote fécal d'un sujet donneur, comprenant les étapes suivantes:
a) le prélèvement ex vivo d'au moins un échantillon de microbiote fécal du sujet donneur,
b) dans un délai inférieur à 5 minutes suivant le prélèvement, le placement dudit échantillon obtenu en a) dans un dispositif de collecte étanche à l'oxygène,
c) le mélange de l'échantillon obtenu en b) avec au moins une solution aqueuse saline comprenant au moins un cryoprotectant choisi parmi le tréhalose, le galactose-lactose et leurs mélanges et un agent de charge,
d) optionnellement, la filtration du mélange obtenu en c), notamment par un filtre comprenant des pores de diamètre inférieur ou égal à 0.7 mm, de préférence inférieur ou égal à 0.5 mm, et
e) le stockage du mélange obtenu en c) ou d) par congélation à une température comprise entre -15°C et -100°C, de préférence entre -60°C et -90°C,
les étapes b) à e) étant réalisées en anaérobiose.

2. Procédé selon la revendication 1, **caractérisé en ce que** l'échantillon placé dans le dispositif de collecte de l'étape b) subit une étape de transport avant l'étape c).

3. Procédé selon l'une des revendications 1 à 2, **caractérisé en ce que** l'échantillon placé dans le dispositif de collecte de l'étape b) est incubé à une température comprise entre 33°C et 40°C pendant une durée maximale de 75h, entre les étapes b) et c).

4. Procédé selon l'une des revendications 1 à 3, **caractérisé en ce que** la quantité totale de cryoprotectant présent dans la solution aqueuse saline est comprise entre 3 et 30% en poids par rapport au volume total de solution, de préférence entre 4% et 20% en poids par rapport au volume total de solution.

5. Procédé selon l'une des revendications 1 à 4, **caractérisé en ce que** la solution aqueuse saline comprend:
- au moins un sel choisi parmi le chlorure de sodium, le chlorure de calcium, le chlorure de magnésium, le chlorure de potassium, le sodium gluconate et le sodium acétate, et
- optionnellement au moins un antioxydant, de préférence choisi parmi le L-ascorbate de sodium, les tocophérols, le chlorhydrate de L-cystéine monohydraté et leurs mélanges.

6. Procédé selon l'une des revendications 1 à 5, **caractérisé en ce que** l'agent de charge est des maltodextrines.

7. Procédé selon l'une des revendications 1 à 6, **caractérisé en ce que** l'agent de charge est un mélange de maltodextrines, dans lequel la quantité de maltodextrines est comprise entre 4 et 20% en poids par rapport au volume total de solution.

8. Procédé selon l'une des revendications 1 à 7, **caractérisé en ce qu'**il comprend une étape f) de décongélation de l'échantillon congelé obtenu en e), en anaérobiose, jusqu'à température ambiante.

9. Echantillon de microbiote fécal d'un sujet donneur susceptible d'être obtenu par le procédé selon la revendication 8, pour son utilisation dans la transplantation de microbiote fécal autologue ou allogénique.

10. Echantillon de microbiote fécal d'un sujet donneur susceptible d'être obtenu par le procédé selon la revendication 8, pour son utilisation pour traiter les dysbioses intestinales.

## Patentansprüche

1. Verfahren zur Herstellung einer Probe der fäkalen Mikrobiota eines Spenders, welches die folgenden Schritte umfasst:
a) Ex-vivo-Entnahme mindestens einer Probe der fäkalen Mikrobiota des Spenders,
b) innerhalb eines Zeitraum von weniger als 5 Minuten nach der Entnahme Platzierung der in a) erhaltenen Probe in einer sauerstoffdichten Sammelvorrichtung,
c) Vermischung der in b) erhaltenen Probe mit mindestens einer wässrigen Salzlösung, die mindestens ein aus Trehalose, Galactose-Lactose und deren Mischungen ausgewähltes Gefrierschutzmittel und einen Füllstoff umfasst,
d) optional Filtration der in c) erhaltenen Mischung, insbesondere durch einen Filter, der Poren mit einem Durchmesser kleiner oder gleich 0,7 mm, bevorzugt kleiner oder gleich 0,5 mm umfasst, und
e) Lagerung der in c) oder d) erhaltenen Mischung durch Einfrieren bei einer Temperatur, die zwischen -15°C und -100°C, bevorzugt zwischen -60°C und -90°C liegt,
wobei die Schritte b) bis e) unter Anaerobiose durchgeführt werden.

2. Verfahren nach Anspruch 1, **dadurch gekennzeichnet, dass** die in der Sammelvorrichtung platzierte Probe aus dem Schritt b) vor dem Schritt c) einem Transportschritt unterzogen wird.

3. Verfahren nach einem der Ansprüche 1 oder 2, **dadurch gekennzeichnet, dass** die in der Sammelvorrichtung platzierte Probe aus dem Schritt b) bei einer Temperatur, die zwischen 33°C und 40°C liegt, während einer Dauer von maximal 75h zwischen den Schritten b) und c) inkubiert wird.

4. Verfahren nach einem der Ansprüche 1 bis 3, **dadurch gekennzeichnet, dass** die Gesamtmenge des in der wässrigen Salzlösung vorhandenen Gefrierschutzmittels zwischen 3 und 30 Gewichtsprozent liegt, bezogen auf das Gesamtvolumen der Lösung, bevorzugt zwischen 4 und 20 Gewichtsprozent, bezogen auf das Gesamtvolumen der Lösung.

5. Verfahren nach einem der Ansprüche 1 bis 4, **dadurch gekennzeichnet, dass** die wässrige Salzlösung umfasst:
- mindestens ein Salz, welches ausgewählt ist aus Natriumchlorid, Calciumchlorid, Magnesiumchlorid, Kaliumchlorid, Natriumgluconat und Natriumacetat, und
- optional mindestens ein Antioxidans, welches bevorzugt ausgewählt ist aus Natrium-L-Ascorbat, Tocopherolen, L-Cysteinhydrochlorid-Monohydrat und deren Mischungen.

6. Verfahren nach einem der Ansprüche 1 bis 5, **dadurch gekennzeichnet, dass** der Füllstoff Maltodextrine ist.

7. Verfahren nach einem der Ansprüche 1 bis 6, **dadurch gekennzeichnet, dass** der Füllstoff eine Mischung von Maltodextrinen ist, in welchem die Menge an Maltodextrinen zwischen 4 und 20 Gewichtsprozent liegt, bezogen auf das Gesamtvolumen der Lösung.

8. Verfahren nach einem der Ansprüche 1 bis 7, **dadurch gekennzeichnet, dass** es einen Schritt f) des Auftauens der in e) erhaltenen gefrorenen Probe unter Anaerobiose bis auf Raumtemperatur umfasst.

9. Probe der fäkalen Mikrobiota eines Spenders, die geeignet ist, durch das Verfahren nach Anspruch 8 erhalten zu werden, zur Verwendung in der autologen oder allogenen Transplantation von fäkaler Mikrobiota.

10. Probe der fäkalen Mikrobiota eines Spenders, die geeignet ist, durch das Verfahren nach Anspruch 8 erhalten zu werden, zur Verwendung für die Behandlung von Darmdysbiosen.

## Claims

1. A method of preparing a sample of faecal microbiota from a donor subject, comprising the following steps:
a) taking *ex vivo* at least one sample of faecal microbiota from the donor subject,
b) within 5 minutes following taking of the sample, placing said sample obtained in a) in an oxygen-tight collecting device,
c) mixing the sample obtained in b) with at least one saline aqueous solution comprising at least one cryoprotectant chosen from trehalose, galactose-lactose and mixtures thereof and one bulking agent,
d) optionally, filtering the mixture obtained in c), in particular using a filter comprising pores of diameter less than or equal to 0.7 mm, preferably less than or equal to 0.5 mm, and
e) storing the mixture obtained in c) or d) by freezing at a temperature between -15°C and -100°C, preferably between -60°C and -90°C,
steps b) to e) being carried out under anaerobiosis.

2. The method according to claim 1, **characterized in that** the sample placed in the collecting device of step b) undergoes a transportation step prior to step c).

3. The method according to one of claims 1 to 2, **characterized in that** the sample placed in the collecting device of step b) is incubated at a temperature comprised between 33°C and 40°C for a maximum time of 75 h, between steps b) and c).

4. The method according to one of claims 1 to 3, **characterized in that** the total amount of cryoprotectant present in the saline aqueous solution is comprised between 3 and 30% by weight relative to the total volume of solution, preferably between 4% and 20% by weight relative to the total volume of solution.

5. The method according to one of claims 1 to 4, **characterized in that** the saline aqueous solution comprises:
- at least one salt chosen from sodium chloride, calcium chloride, magnesium chloride, potassium chloride, sodium gluconate and sodium acetate, and
- optionally at least one antioxidant, preferably chosen from sodium L-ascorbate, tocopherols, L-cysteine hydrochloride monohydrate and mixtures thereof.

6. The method according to one of claims 1 to 5, **characterized in that** the bulking agent is maltodextrins.

7. The method according to one of claims 1 to 6, **characterized in that** the bulking agent is a mixture of maltodextrins, in which the amount of maltodextrins is comprised between 4 and 20% by weight relative to the total volume of solution.

8. The method according to one of claims 1 to 7, **characterized in that** it comprises a step f) of thawing the frozen sample obtained in e), under anaerobiosis, up to ambient temperature.

9. A sample of fecal microbiota from a donor subject obtainable by the method according to claim 8, for use in the transplantation of autologous or allogenic faecal microbiota.

10. The sample of fecal microbiota from a donor subject obtainable by the method according to claim 8, for use in treating intestinal dysbioses.
